# DEMANDE DE BREVET EUROPEEN

(11) **EP 1 462 140 A1**
(43) Date de publication de la demande: **29.09.2004**
(21) Numéro de dépôt: 04300158.5
(22) Date de dépôt: 23.03.2004
(51) Int. Cl.: A61M 16/12

(54) **Ensemble portatif de ventilation d'urgence**

(30) Priorité: 26.03.2003 FR 0303716
(71) Demandeur: TAEMA, F-92182 Antony Cédex (FR)
(72) Inventeur: Bleys, Christian, 77000 Livry s/Seine (FR); Kissi, Noureddine, 92360 Meudon La Forêt (FR)
(74) Mandataire: Pittis, Olivier

(57) **Abrégé**

L'invention porte sur un ensemble portatif de ventilation d'urgence comprenant une source (1) de gaz sous pression équipée d'un dispositif robinet-détendeur (2) de gaz permet de contrôler le débit et/ou la pression du gaz sortant de la source de gaz (1), un ventilateur (3) d'assistance respiratoire alimenté en gaz par ladite source (1) de gaz, et une interface homme/machine (4) coopérant avec ledit ventilateur (3) de manière à permettre un réglage d'au moins un paramètre ventillatoire et/ou d'au moins une consigne de ventilation. L'ensemble portatif de l'invention constitue un matériel de secours ventillatoire, utilisable dans des situations d'urgence, qui est d'encombrement réduit et de faible poids, lequel est apte à être transporté facilement par un médecin ou analogue, y compris dans des endroits difficiles d'accès.

## Description

La présente invention concerne un ensemble portatif de ventilation d'urgence comprenant une bouteille d'oxygène, un mini-ventilateur alimentant un circuit patient se terminant par un masque respiratoire ou analogue, et une interface homme-machine coopérant avec le ventilateur.

Actuellement, lorsqu'un médecin d'un service d'urgences intervient pour secourir un patient en détresse respiratoire, il est habituellement équipé d'une source d'oxygène, par exemple une bouteille oxygène comprimé, et d'un appareil d'urgence qui est relié à la fois à la source de gaz par un tuyau flexible et au patient par un tuyau et un masque respiratoire.

Or, ces matériels sont lourds et encombrants, ce implique que leur transport par le médecin nécessite généralement l'usage de ses deux mains.

Si le lieu d'intervention est difficile d'accès, par exemple un site escarpé, une galerie étroite ou un lieu exigu, l'acheminement de tout le matériel doit souvent se faire en plusieurs étapes, c'est-à-dire plusieurs allers-retours, ou requière plusieurs personnes.

Par ailleurs, pour l'installation et les réglages, la mise en oeuvre est longue car le médecin doit d'abord opérer toutes les connexions ou autres raccordements, puis se consacrer aux réglages de la machine d'urgence, du débit et/ou de la pression du gaz délivré par la bouteille, le mode ventillatoire, par exemple ventilation contrôlée (VC) ou ventilation assistée contrôlée (VAC), la pression expiratoire positive (PEP), le seuil de déclenchement inspiratoire, la fréquence respiratoire, le rapport du temps inspiratoire sur le temps expiratoire, la pression maximale de sécurité et d'autres paramètres de ventilation ou de sécurité et ce, avant de pourvoir s'occuper spécifiquement de la victime.

On comprend dès lors que, dans certains cas extrêmes, le temps nécessaire pour toutes ces opérations peut être fatal à la victime.

Le problème qui se pose alors dans ce contexte est de pouvoir disposer d'un matériel de secours ventilatoire, utilisable dans les situations d'urgence, qui soit d'encombrement réduit et de faible poids, lequel soit apte à être transporté facilement par un médecin ou analogue, y compris dans des endroits difficiles d'accès, et ce, en ne nécessitant que l'usage d'une seule main dudit médecin, voire d'aucune lorsqu'il est placé dans un dispositif de portage adapté.

La solution de l'invention est alors un ensemble portatif de ventilation d'urgence comprenant :
- une source de gaz sous pression équipée d'un dispositif robinet-détendeur de gaz permet de contrôler le débit et/ou la pression du gaz sortant de la source de gaz,
- un ventilateur d'assistance respiratoire alimenté en gaz par ladite source de gaz, et
- une interface homme/machine coopérant avec ledit ventilateur de manière à permettre un réglage d'au moins un paramètre ventillatoire et/ou d'au moins une consigne de ventilation.

Selon le cas, l'ensemble de l'invention peut comprendre l'une ou plusieurs des caractéristiques techniques suivantes :
- le dispositif robinet-détendeur de gaz comporte un raccord de sortie sur lequel vient se fixer le ventilateur d'assistance respiratoire.
- le ventilateur d'assistance respiratoire comprend un circuit interne de gaz reliant fluidiquement un orifice d'entrée à un orifice de sortie, une vanne proportionnelle étant agencée sur ledit circuit interne, ladite vanne étant commandée par des moyens de pilotage coopérant avec l'interface homme-machine.
- le ventilateur d'assistance respiratoire comprend, par ailleurs, un injecteur à venturi agencé sur le circuit interne, en aval de la vanne proportionnelle.
- le ventilateur d'assistance respiratoire comprend, en outre, un capteur de débit et un capteur de pression pour mesure le débit et la pression du gaz dans le circuit interne, lesdits capteurs coopérant avec les moyens de pilotage de manière à permettre un asservissement et une régulation de la vanne proportionnelle en débit et/ou en pression.
- l'interface homme-machine comporte des moyens de réglage de consigne ou de paramètre de ventilation pour permettre une sélection et/ou un réglage d'au moins un paramètre ventillatoire et/ou d'au moins une consigne de ventilation et préférentiellement des moyens d'affichage coopérant avec lesdits moyens de réglage pour permettre de visualiser et/ou d'afficher au moins une valeur d'au moins un paramètre ventillatoire et/ou d'au moins une consigne de ventilation sélectionné et/ou réglé.
- il comporte un circuit patient comprenant au moins un conduit de gaz se raccordant, par son extrémité amont, au niveau de l'orifice de sortie du ventilateur et, par son extrémité aval, à un masque respiratoire.
- le robinet-détendeur et le ventilateur sont protégés par un capotage de protection venant se fixer sur la source de gaz.
- les moyens de réglage de consigne ou de paramètre de ventilation permettent une sélection et/ou un réglage d'au moins un paramètre ventillatoire et/ou d'au moins une consigne de ventilation choisi dans le groupe formé par la fréquence ventillatoire, le débit de ventilation, le volume de ventilation, la composition du mélange gazeux, le seuil de déclenchement inspiratoire, le temps inspiratoire et/ou le temps expiratoire ou leur rapport, la pression expiratoire positive (PEP), le mode ventillatoire et la pression maximale de sécurité.
- le dispositif robinet-détendeur, le ventilateur d'assistance respiratoire et l'interface homme/machine forment un système compact porté par la source de gaz, en particulier par une bouteille d'oxygène.
- il a un poids total inférieur à 25 kg, de préférence inférieur à 15 kg, de manière à pouvoir être porté facilement par un médecin.
- il est agencé dans un moyen de portage, tel qu'un sac à dos, un harnais de portage ou tout moyen de portage similaire.

L'invention va être décrite plus en détail en références aux figures annexées, données à titre illustratif.

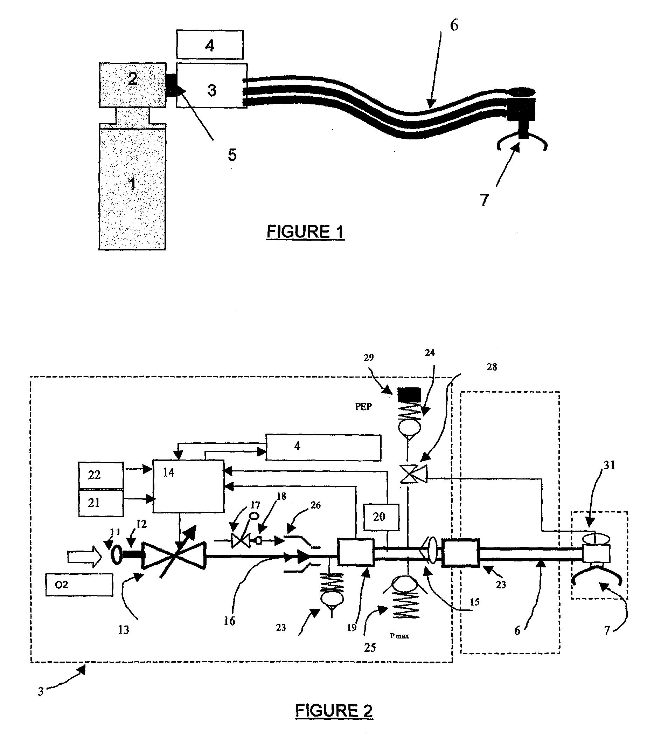

Comme schématisé sur la Figure 1, l'ensemble de l'invention est composé de trois éléments principaux, à savoir une source 1 de gaz, en particulier une bouteille d'oxygène, munie d'un robinet à détendeur intégré 2 avec une sortie basse pression normalisée, et un micro ventilateur 3 ayant, côté entrée de gaz, un raccord d'entrée normalisé basse pression permettant le montage direct sur la sortie 5 du robinet-détendeur 2 équipant la source 1 de gaz, typiquement une bouteille d'oxygène.

Le micro ventilateur 3 peut par exemple être monté sur une bouteille 1 de gaz munie d'un capotage de protection, tel que décrit par les documents EP-A-629812 ou EP-A-811900.

Les éléments composant le ventilateur 3, représentés sur la Figure 2, sont de taille réduite, ce qui permet de les loger dans un volume restreint.

Ainsi, sur la Figure 2, on voit que le micro-ventilateur 3 d'assistance respiratoire comprend un orifice d'entrée 11 de gaz venant se raccorder sur le raccord de sortie 5 basse pression du robinet-détendeur 2, et un orifice de sortie 15 de gaz par lequel le gaz est envoyé vers le circuit patient 6, le gaz étant acheminé de l'orifice d'entrée 11 à l'orifice de sortie 15 par un circuit interne 12 d'acheminement de gaz sur lequel est agencée une vanne proportionnelle 13 commandée par des moyens de pilotage 14 comportant une carte électronique. La vanne proportionnelle 13 permet de réguler la proportion de gaz envoyé vers le circuit patient 6.

Le circuit interne 12 comprend aussi, en aval de la vanne proportionnelle 13, un ensemble éjecteur pouvant réaliser un mélange d'air et d'oxygène dans des proportions volumiques variables entre 60 et 100% d'oxygène. Cet ensemble comprend un injecteur 16 de gaz et un convergent droit, en particulier du type à venturi, relié à l'atmosphère ambiant par l'intermédiaire d'un clapet anti-retour 18 et un distributeur mécanique à commande manuelle 17 ayant au moins deux positions et au moins deux orifices, à savoir notamment une position correspondant à une ventilation à 100% d'oxygène (O₂ pur sans apport d'air ambiant) et une position correspondant à une ventilation avec mélange air/oxygène.

Le clapet anti-retour 18 évite l'échappement de l'oxygène en cas de contre pression importante dans le circuit patient 6.

Le distributeur 17 permet le passage de l'air atmosphérique vers le convergent/divergent 26 pour réaliser le mélange air oxygène ou, à l'inverse, pour ne pas réaliser ce mélange lorsqu'il est nécessaire de ventiler le patient avec de l'oxygène pur.

Des capteurs de débit 19 et pression 20 sont reliés au circuit interne 12, en aval du de l'injecteur 16 à venturi de manière à pouvoir mesurer le débit et la pression du gaz dans le circuit interne 12 en aval de l'injecteur 16.

Grâce à la vanne proportionnelle 13 associée à l'entrée de l'injecteur 16 à venturi et aux capteurs 19, 20 débit et de pression, il est possible de gérer la commande d'ouverture et de fermeture de ladite vanne 13 en fonction des consignes de débit et pression réglées par le médecin sur l'interface homme/machine 4 et en réponse aux mesures de débit et pression opérées par les capteurs 19, 20 qui coopèrent avec les moyens de pilotage 14.

En effet, le micro-ventilateur 3 incorpore également une interface homme/machine 4 permettant au médecin d'effectuer tous les réglages très simplement.

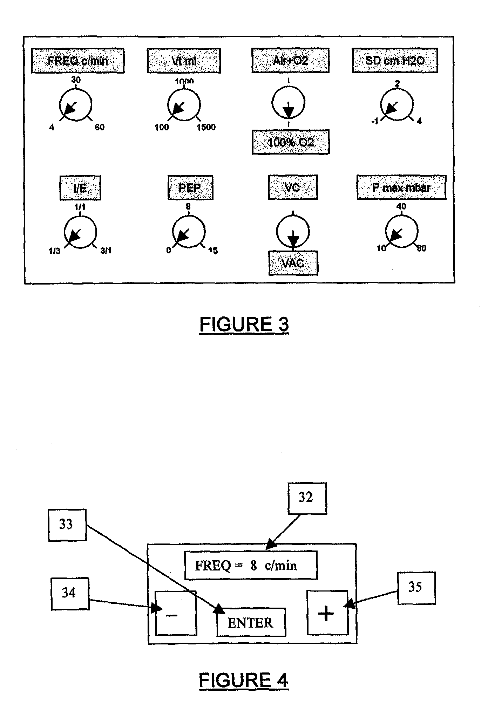

Cette interface homme/machine 4 se compose par exemple, comme montré en figure 3, d'un moyen de réglage de fréquence (FREQ c/min), d'un moyen de réglage (Vt ml) de débit de ventilation minute ou de volume courant, d'un moyen de commande à deux positions (position air + oxygène, et position oxygène pur), d'un moyen de réglage du seuil de déclenchement inspiratoire (SD cm H2O), d'un moyen de réglage du temps inspiratoire et du temps expiratoire ou le rapport des deux (I/E), d'un moyen de réglage de la pression expiratoire positive (PEP), d'un moyen de réglage du mode ventilatoire (VCNAC) et d'un moyen de réglage de la pression maximale de sécurité (P max mbar). Ces différents moyens sont par exemple des boutons rotatifs ou tout système d'actionnement similaire ou équivalent.

Pour réduire l'encombrement de l'interface homme/machine 4, on peut remplacer le système schématisé en figure 3 par un système de réglage du type à affichage des différents paramètres de ventilation mesurés par la machine de manière à pouvoir également informer le médecin du bon déroulement de l'intervention et accroître encore la sécurité du patient.

Un tel système est schématisé en Figure 4 où l'on voit qu'il comporte un moyen d'affichage 32 d'information, tel un écran à cristaux liquides ou analogue, permettant de visualiser les différents paramètres à régler susmentionnés (fréquence, débit, PEP....).

La sélection de ces paramètres à régler se fait par l'intermédiaire de moyens de sélection 33, 34, 35 comportant ici plusieurs boutons ou touches de sélections actionnables digitalement par le médecin, à savoir des touches + et - de sélection 34, 35 de paramètre ou de fonction et une touche 33 de validation du paramètre ou de la fonction choisie.

Le réglage de la valeur souhaitée de chaque paramètre s'effectue également avec les boutons ou touches 34, 35 de sélection (touches + et -) et la validation de la valeur choisie par actionnement de la touche de validation 33 (touche "entrée", i.e. "ENTER") ; à noter toutefois, que d'autres touches spécifiques peuvent être également prévues à ces fins.

Comme montré sur la Figure 2, les moyens de pilotage 14 son alimentés en courant électrique par une source de courant 22, telle une batterie rechargeable, des piles ou analogues, et peut aussi comprendre une prise de raccordement électrique au secteur 21.

Le micro-ventilateur 3 comporte aussi un système de réglage de la PEP (pression expiratoire positive) comprenant un bouton de réglage 29 et un ensemble 24 constitué d'un siège, d'une membrane sur laquelle repose un moyen à ressort qui est plus ou moins comprimé par le bouton 29. Ladite membrane obture plus ou moins l'orifice qui relie le circuit patient et l'atmosphère par l'intermédiaire de l'électrovanne 28 pendant la phase expiratoire.

Par ailleurs, la soupape 25 permet de mettre le circuit patient à l'atmosphère si la pression de ventilation patient venait à atteindre la pression maximale critique dans le circuit patient 6.

Autrement dit, le système de réglage de la PEP 24 comprend un orifice relié, par une extrémité, au circuit qui débouche sur le circuit patient 6 qui alimente le patient en gaz et qui se trouve en aval de l'ensemble 26 et communiquant , via son autre extrémité, avec l'atmosphère lorsque la pression dans le conduit 6 devient supérieure à la force de pression réglable qu'exerce un moyen élastique 24, tel un ressort, sur une membrane ou analogue agencé au niveau de ladite extrémité communiquant avec l'atmosphère.

Une électrovanne expiratoire 28 ayant trois orifices et deux positions étant agencée entre le circuit patient 6, l'ensemble PEP 24 et la commande de la valve expiratoire 31.

En phase inspiratoire, l'électrovanne 28 prélève la pression générée par la machine pour pressuriser le ballonnet de la valve expiratoire de façon à isoler le patient de l'atmosphère et permettre au fluide d'alimenter le patient via le masque 7 ou une sonde d'intubation.

En phase expiratoire, l'électrovanne 28 isole la commande de la valve expiratoire 31 de la pression patient et la relie à l'ensemble PEP 24 pour permettre au patient d'expirer à la pression atmosphérique ou la pression expiratoire positive réglée.

De façon analogue, le système de sécurité 25 de la pression maximale autorisée dans le circuit interne 6 comprend un conduit relié, d'une part, au circuit interne 6 et, d'autre part, à l'atmosphère. En cas de montée de pression excessive dans le circuit 6, l'ensemble 25 permet le passage du gaz depuis le circuit 6 vers l'extérieur. Comme précédemment l'ensemble 25 étant contrôlé par un clapet sur lequel s'exerce une force de pression exercée par un moyen élastique type ressort. Le clapet anti-retour 15 isole la machine du patient dans le cas où ce dernier refoule le gaz expiré vers la machine.

La sortie du micro ventilateur est équipée d'un raccord de sortie par exemple en forme de cône de 22 mm normalisé pour le raccordement du circuit patient 6 auquel est raccordé un masque respiratoire 7.

L'ensemble de l'invention peut être disposé dans un sac à dos de portage permettant à l'utilisateur de garder toute sa liberté de mouvements pour accéder au lieu d'accident.

Autrement dit, la solution de l'invention est basée sur un ensemble très compact composé d'une bouteille d'oxygène équipée d'un robinet détendeur intégré sur lequel un ventilateur d'urgence de taille très réduite est connecté directement sur la sortie basse pression normalisée. L'olive de sortie débit du robinet reste libre pour l'utilisation éventuelle d'une lunette d'oxygénothérapie dans le cadre d'une moindre urgence.

Le micro-ventilateur d'urgence connecté directement sur la prise est ainsi au plus prêt de la source et en partie protégé par le chapeau de protection du robinet de la bouteille, lorsque la bouteille est équipée d'un tel capotage de protection.

La fixation du micro-ventilateur peut être renforcée par des moyens supplémentaires, telles des sangles ou autres attaches rapides.

La mise en oeuvre de la bouteille ne nécessite qu'un seul geste d'ouverture par rotation de ¼ de tour de la commande.

Pour la mise en oeuvre de l'ensemble, le médecin ou analogue a très peu de gestes à réaliser une fois sur le lieu d'utilisation, à savoir notamment :
- il doit ouvrir le robinet de la bouteille (¼ tour),
- mettre en route le ventilateur par actionnement d'une commande de marche/arrêt,
- régler en fonction du patient la fréquence respiratoire, le rapport du temps inspiratoire sur le temps expiratoire au moyen de l'interface homme/machine,
- choisir la ventilation en oxygène pur ou ventilation en oxygène mélangé avec de l'air au moyen de l'injecteur 16 de gaz, la consigne du débit insufflé ou le volume courant administré au patient, et le mode ventilatoire adéquat,
- ajuster le seuil de déclenchement de ventilation de la machine.

Le micro-ventilateur 3 est alors alimenté directement par l'orifice de sortie de la bouteille 1 sur laquelle il est connecté de façon solidaire, la sortie du micro-ventilateur 3 étant, quant à elle, reliée directement au circuit patient 6 à l'extrémité duquel se trouve le masque respiratoire 7 avec une valve expiratoire 31.

## Revendications

1. Ensemble portatif de ventilation d'urgence comprenant :
- une source (1) de gaz sous pression équipée d'un dispositif robinet-détendeur (2) de gaz permet de contrôler le débit et/ou la pression du gaz sortant de la source de gaz (1),
- un ventilateur (3) d'assistance respiratoire alimenté en gaz par ladite source (1) de gaz, et
- une interface homme/machine (4) coopérant avec ledit ventilateur (3) de manière à permettre un réglage d'au moins un paramètre ventillatoire et/ou d'au moins une consigne de ventilation.

2. Ensemble selon la revendication 1, **caractérisé en ce que** le dispositif robinet-détendeur (2) de gaz comporte un raccord de sortie (5) sur lequel vient se fixer le ventilateur (3) d'assistance respiratoire.

3. Ensemble selon l'une des revendications 1 ou 2, **caractérisé en ce que** le ventilateur (3) d'assistance respiratoire comprend un circuit interne (12) de gaz reliant fluidiquement un orifice d'entrée (11) à un orifice de sortie (23), une vanne proportionnelle (13) étant agencée sur ledit circuit interne (12), ladite vanne (13) étant commandée par des moyens de pilotage (14) coopérant avec l'interface homme-machine (4).

4. Ensemble selon l'une des revendications 1 à 3, **caractérisé en ce que** le ventilateur (3) d'assistance respiratoire comprend, par ailleurs, un injecteur (16) à venturi agencé sur le circuit interne (12), en aval de la vanne proportionnelle (13).

5. Ensemble selon l'une des revendications 1 à 4, **caractérisé en ce que** le ventilateur (3) d'assistance respiratoire comprend, en outre, un capteur de débit (19) et un capteur de pression (20) pour mesure le débit et la pression du gaz dans le circuit interne (12), lesdits capteurs coopérant avec les moyens de pilotage (14) de manière à permettre un asservissement et une régulation de la vanne proportionnelle (13) en débit et/ou en pression.

6. Ensemble selon l'une des revendications 1 à 5, **caractérisé en ce que** l'interface homme-machine (4) comporte des moyens de réglage (33, 34, 35) de consigne ou de paramètre de ventilation pour permettre une sélection et/ou un réglage d'au moins un paramètre ventillatoire et/ou d'au moins une consigne de ventilation et préférentiellement des moyens d'affichage (32) coopérant avec lesdits moyens de réglage pour permettre de visualiser et/ou d'afficher au moins une valeur d'au moins un paramètre ventillatoire et/ou d'au moins une consigne de ventilation sélectionné et/ou réglé.

7. Ensemble selon l'une des revendications 1 à 6, **caractérisé en ce qu'**il comporte un circuit patient (6) comprenant au moins un conduit de gaz se raccordant, par son extrémité amont, au niveau de l'orifice de sortie (23) du ventilateur et, par son extrémité aval, à un masque respiratoire (7).

8. Ensemble selon l'une des revendications 1 à 7, **caractérisé en ce que** le robinet-détendeur (2) et le ventilateur (3) sont protégés par un capotage de protection venant se fixer sur la source de gaz (1).

9. Ensemble selon l'une des revendications 1 à 8, **caractérisé en ce que** les moyens de réglage (33, 34, 35) de consigne ou de paramètre de ventilation permettent une sélection et/ou un réglage d'au moins un paramètre ventillatoire et/ou d'au moins une consigne de ventilation choisi dans le groupe formé par la fréquence ventillatoire, le débit de ventilation, le volume de ventilation, la composition du mélange gazeux, le seuil de déclenchement inspiratoire, le temps inspiratoire et/ou le temps expiratoire ou leur rapport, la pression expiratoire positive (PEP), le mode ventillatoire et la pression maximale de sécurité.

10. Ensemble selon l'une des revendications 1 à 9, **caractérisé en ce que** le dispositif robinet-détendeur (2), le ventilateur (3) d'assistance respiratoire et l'interface homme/machine (4) coopérant avec ledit ventilateur (3) forment un système compact porté par la source de gaz (1), en particulier par une bouteille d'oxygène.
